# EUROPÄISCHE PATENTSCHRIFT

(11) **EP 2 062 482 B1**
(45) Veröffentlichungstag und Bekanntmachung des Hinweises auf die Patenterteilung: **04.04.2012**
(21) Anmeldenummer: 08020141.1
(22) Anmeldetag: 02.11.2004
(51) Int. Cl.: A23L 1/304

(54) **Zinkhaltiges Nahrungsergänzungsmittel für Mensch und Tier**
Food additive containing zinc for people and animals
Complément alimentaire contenant du zinc pour humains et animaux

(30) Priorität: 04.11.2003 DE 10352129
(43) Veröffentlichungstag der Anmeldung: 27.05.2009
(62) Teilanmeldung aus: 04025917.8
(73) Patentinhaber: Grillo Zinkoxid GmbH, 38644 Goslar (DE)
(72) Erfinder: Jahn,Burkhardt, Dr. rer. nat. Dip.-Chem., VERSTORBEN (DE); Schubert, Peter, Dipl.-Chem., 38690 Vienenburg (DE)
(74) Vertreter: Lins, Martina

(56) Entgegenhaltungen:
- CA-A1- 2 445 708
- DE-A1- 2 436 946
- AKIHAMA S ET AL: "ANTIVIRAL EFFECT OF ZINC COMPLEXES ON JAPANESE B ENCEPHALITIS VIRUS" CHEMICAL AND PHARMACEUTICAL BULLETIN, PHARMACEUTICAL SOCIETY OF JAPAN, TOKYO, JP, Bd. 10, Nr. 12, 1. Januar 1962 (1962-01-01), Seiten 1254-1261, XP008042423 ISSN: 0009-2363

## Beschreibung

Die Erfindung betrifft allgemein die Verwendung einer Organozinkverbindung zusammen mit Zinkoxid in einem Nahrungs- oder Futter-Ergänzungsmittel, nämlich ein zinkhaltiges Nahrungsergänzungsmittel für Mensch und Tier, enthaltend eine wenigstens vierfach organisch koordinierte oder zweifach organisch substituierte Zinkverbindung und einen Anteil an Zinkoxid, sowie die Verwendung von Zinkoxid in Verbindung mit wenigstens einem Aminosäure-Zink-Komplex in einem Nahrungs- oder Futter-Ergänzungsmittel. Es wird ein Verfahren zur Herstellung der in dem Mittel enthaltenen Organozinkverbindung angegeben.

Seit langem ist bekannt, dass das Element Zink für die Entwicklung und Gesunderhaltung von Pflanzen, Tieren und Menschen wesentlich ist. Zink aktiviert verschiedene Enzyme und ist Bestandteil vieler wichtiger Metalloenzyme; es ist daher ein wesentliches intrazelluläres Spurenelement.

Besonders in der industriellen Massentierhaltung werden Spurenelemente und Mineralien als Futterzusätze für eine ausgewogene Ernährung und ein gutes Gedeihen der Tiere benötigt. Spezielle Futterzusammensetzungen, beispielsweise mit hohem Kalziumgehalt, können allerdings die Bioverfügbarkeit von Zink in der Nahrung beeinträchtigen. Ebenso kann die Zinkzufuhr bei Diäten von Mensch und Tier zu gering sein. Einzeln oder im Gemisch mit anderen Zusatzstoffen werden daher seit längerer Zeit Zinkverbindungen für die Nahrungsergänzung von Mensch und Tier gegeben.

Als Nahrungs- oder Futter-Ergänzungsmittel sind sowohl anorganische wie auch organische Zinkverbindungen im Gebrauch. Unter anderem werden Zinkoxid (ZnO), Zinksulfat (ZnSO4, i.a. als Hydrat), Zinkchlorid (ZnCl2) und organische Zinkverbindungen verwendet. Von organischen Salzen erhofft man sich im Allgemeinen eine höhere Bioverfügbarkeit. Speziell sind auch Aminosäure-Zink-Komplexe bekannt. Dies bringt den Vorteil mit sich, dass gemeinsam sowohl Zink als auch Aminosäure verabreicht werden können, da den Futterzusatzstoffen ohnehin vielfach Aminosäuren oder Aminosäuregemische beigefügt sind. In der Tierernährung verwendete Aminosäuren sind vor allem: Methionin,

Histidin, Phenylalanin, Lysin, Thryptophan, Threonin, Isoleucin, Valin, Arginin und Leucin (essentielle Aminosäuren für Mensch, Geflügel, Schwein).

Für die Futtermittelergänzung sind unter anderem Zink-Aminosäure 1:1-Komplexe bekannt, wie beispielsweise Zinkmethionin- oder Zinklysin-Komplexe sowie Aminosäuregemisch-Zink-Verbindungen. Auch als Zinkgluconat wird Zink häufig gegeben, und zwar für Mensch und Tier.

Aus der DE 24 36 946 A1 ist die Herstellung von Zink-Methionin 1:1-Komplexsalzen der Form Zink(methionato-N,O)Xn bekannt, wobei X für ein beliebiges ein- oder zweiwertiges Anion steht (n=1;2), vorzugsweise Chlorid, Hydrogensulfat oder Acetat.

Die CA-A1-2 445 708 (Japan Science & Tech Agency, JP) vom 14. November 2002 offenbart ein Nahrungsergänzungsmittel, das unter anderem eine OrganoZinkverbindung und Zinkoxid in der Form 5 ZnO • 2CO₂ • 4 H₂O vorgemischt innerhalb eines Mineral-Premix enthält.

Die US 3,463,858 A offenbart ein Nahrungsergänzungsmittel mit einem anorganischen Zinkwachstumsfaktor für die Tiernahrung und ein nasschemisches Verfahren zur Herstellung des Zinkkomplexes aus einer Aminosäurequelle und einem wasserlöslichen Zinksalz durch Erwärmen in Wasser, Ansäuern und Trocknen des Rückstandes. Das Mittel kann u.a. ein Spurenelementgemisch enthalten, das wiederum ZnO für das Spurenelement Zn enthalten kann.

Die Herstellung erfolgt, indem die gewünschte Aminosäure, hier Methionin, einer heißen Lösung eines anorganischen Zinksalzes zugegeben wird, oder indem ein Hydratsalz mit Methionin erwärmt wird.

Aus der Stammanmeldung EP 1 529 774 A2 ist ein Verfahren bekannt, bei dem als Zinkverbindung Zinkoxid eingesetzt wird, das Zinkoxid und die Säure gemischt werden, das Gemisch in Substanz, ohne Zugabe von Lösungsmittel durch Energiezufuhr mittels Wärme oder Bestrahlung auf eine Temperatur unterhalb der Zersetzungstemperatur der Säure, generell bis auf wenigsten 150 °C (gut unterhalb der Zersetzungstemperatur organischer Säuren), erwärmt und für die Reaktionszeit bei oder unterhalb dieser Temperatur gehalten wird.

Vorzugsweise werden organische Säuren verwendet, die bei Normaldruck bis zur Zersetzungstemperatur im festen Aggregatszustand vorliegen, so dass das Verfahren in fester Phase, d.h. im trockenen Gemisch, durchgeführt wird.

Ein Vorteil des Verfahrens liegt darin, dass Zinkoxid industriell in ausreichendem Maßstab und in geeigneter Reinheit zur Verfügung steht. Für verschiedene Anwendungszwecke ist hochreines Zinkoxid in pulverisierter Form erhältlich. Diese Zinkoxide erfüllen die Reinheitsanforderungen nationaler und internationaler Pharmakopeen wie USP, DAB 10 etc. und die der europäischen Futtermittelverordnung. Wegen mangelnder Löslichkeit des Zinkoxids in Wasser kann jedoch die Umsetzung von ZnO mit organischen Säuren zu Salzen dieser organischen Säuren oder zu Organometallkomplexsalzen nicht sinnvoll in Lösung durchgeführt werden.

Überraschenderweise war gefunden worden, dass bei geeigneter Temperaturführung Zinkoxid und organische Säuren in der Feststoffphase, also trocken umgesetzt werden können, ohne dass in einem für ein Nahrungsergänzungsmittel störenden Umfang Neben- oder Zersetzungsprodukte entstünden. Es konnte gezeigt werden, dass dabei ein Säure-Zink 2:1-Komplex oder ein zweiwertig (durch 2 einwertige oder 1 zweiwertiges Säureanion) organisch substituiertes Zinksalz erhalten wird.

Für die Aminosäure-Komplexe wird derzeit davon ausgegangen, dass sich ein "bis({Name der Aminosäure}ato-N,O)-Zink-Komplex bildet, oder allgemein ein binärer Komplex des zweifach positiv geladenen Zinkkations mit zwei Aminosäureanionen, vermutlich über den Sauerstoff der Carboxylatgruppen und den Stickstoff der Aminogruppen gebunden Diese Form des 2:1-Komplexes ist für Methionin belegt (CAS Registrierungsnummer 151214-86-7 für Zink,bis(D-methioninato-N,O)-, (T-4)-(9Cl)). Bei Einsatz optisch reiner Aminosäuren werden auch die entsprechenden Komplexe gefunden. Da als Futtermittelzusatz üblicherweise DL-Methionin gegeben wird, erhielte man einen bis(DL-methioninato-N,O)-Zink-Komplex. Es ist nicht ausgeschlossen, dass sich in der Feststoffphase auch andere Mischformen bilden könnten, z. B. ein Methioninato (methioninato-N,O,S)-Zink-Komplex, bei dem das erste Methionin nur einfach über die Carboxylatgruppe koordiniert ist. Auch polymere Komplexe sind möglich. (Zur Struktur von Aminosäure-Zink-Komplexen, siehe: M. Rombach et al. "Coordination modes of aminoacids to Zinc", Inorganica Chimica Acta, 334 (2002) 25-33).

Die Komplexierung als solche wurde durch die Veränderung der Carbonylschwingung der Carboxylgruppe (²³COO⁻) in dem erfindungsgemäß erhaltenen Verfahrensprodukt nachgewiesen.

Für andere 2:1-Verbindungen aus organischer Säure und Zink, wie sie nach dem Verfahren erhalten werden, sind verschiedene Strukturen möglich. Es kann sich um Carbonsäure-Salze handeln, bei denen je ein Zinkkation mit zwei Carboxylatanionen stöchiometrisch gebunden oder substituiert ist. Die Bindung kann über eine ionische oder vorwiegend ionische Wechselwirkung oder ebenfalls chelatisiert erfolgen, wobei die Carboxylat-Gruppe als bidentale Gruppe symmetrisch über beide Sauerstoffatome mit dem Zink koordiniert sein kann, wie im Zinkacetat (Zn(OOC-CH₃)₂) 2H₂O. Auch polymere Brückenstrukturen sind möglich.

Generell könnte die Reaktion mit ZnO mit folgender Summenformel beschrieben werden:

2 YH + ZnO ZnY₂ + H₂O (Y = (Carbon-)Säureanion)

Es können eine oder mehrere organische Säuren im Gemisch verwendet werden. Vorzugsweise wird wenigstens eine der folgenden Säuren eingesetzt: Methionin, Glycin, Lysin, Leucin, Cystein, Phenylalanin, Histidin, Valin, Prolin, Tryptophan, Isoleucin, Arginin, Asparagin, Thyrosin, Threonin, Weinsäure, Citronensäure, Oxalsäure, Succinsäure, Adipinsäure, Gluconsäure, Alanin, Serin, Cystein, Glutamin, Glutaminsäure, Asparaginsäure, Malonsäure, Maleinsäure, Fumarsäure, jegliche Aminosäuren aus durch Hydrolyse aufgespaltenem Sojaprotein.

Für die Verwendung als Nahrungs- oder Futtermittelzusatz ist es als besonders vorteilhaft anzusehen, dass solche Säuren verwendet werden, die dem Futter/der Nahrung ohnehin zugesetzt werden sollen, also insbesondere Aminosäuren, einzeln oder im Gemisch, wobei Methionin und/oder Lysin besonders bevorzugt sind.

Das Zinkoxid wird als feinkristalline Substanz mit spezifischen Oberflächen von 1 bis 100 m²/gr eingesetzt wird, bevorzugt ZnO-Pulver mit einer BET-Oberfläche von 4 bis 10 m²/gr eingesetzt.

Das in EP 1 529 774 A2 beschriebene Verfahren wird allgemein so durchgeführt, dass das pulverförmige Zinkoxid und die ebenfalls vorzugsweise pulverförmige organische Säure zunächst gemischt werden. Die Reaktion wird in Substanz, d.h. ohne Zusatz von Lösungsmittel durchgeführt. Andere Zusatzstoffe, die physikalisch in das Nahrungs- bzw. Futter-Ergänzungsmittel mit eingebunden werden sollen, können dem Gemisch zusätzlich beigegeben werden, soweit sie in dem für die Reaktion erforderlichen Temperaturbereich stabil sind. Dem Gemisch aus allen Edukten wird nun Energie zugeführt, um die Reaktion zwischen Zink und Säure auszulösen. Vor der Energiezufuhr kann das Gemisch zusätzlich durch Rütteln oder entsprechende Maßnahmen verdichtet werden. Die Energiezufuhr kann durch Wärme oder Bestrahlung erfolgen, dabei wird das Reaktionsgut vorzugsweise in Bewegung gehalten.

Die Bestrahlung erfolgt vorzugsweise mit Mikrowellen. Die Anwendung von Infrarot erscheint möglich, ist jedoch derzeit nicht bevorzugt.

Alternativ kann der Eduktansatz in einem konventionellen Wärmetauscher erwärmt werden, oder das trockene Gemisch kann in einem Drehrohrofen behandelt werden. Dem Fachmann sind Möglichkeiten zur schonenden Erwärmung trockener Gemische hinlänglich bekannt.

Das Verfahren wird so geführt, dass die Energie temperaturgesteuert zugeführt wird. In einer ersten Aufwärmphase wird vorzugsweise konstant(ΔE/t = const.) Energie zugeführt. Es wird jedoch bis maximal 5 °C, vorzugsweise nur bis maximal 10 °C unter die Zersetzungstemperatur der Säure erwärmt, d.h. allgemein nur bis zu einer solchen Temperatur, dass eine Zersetzung der Säure in nennenswertem Ausmaß ausgeschlossen werden kann. Vorzugsweise erfolgt eine Erwärmung auf wenigstens 150 °C, weiter vorzugsweise wenigstens 170 °C, im nachfolgenden Ausführungsbeispiel um 175 °C. Die Temperatur wird dann über einen bestimmten Zeitraum bei gedrosselter Energiezufuhr oder ggf. auch unter Kühlung oder zeitweiser Kühlung konstant gehalten. Auf eine möglichst gleichmäßige Erwärmung über den gesamten Ansatz, d.h. das gesamte Reaktionsvolumen, ist zu achten.

Nach der bemessenen Reaktionszeit wird das Reaktionsgemisch ohne Zufuhr weiterer Energie oder unter Kühlung abkühlen gelassen. Der Fachmann wird das Verhältnis von Maximaltemperatur zu Reaktionsdauer unter anderem unter Berücksichtigung des gewünschten Umsatzes bestimmen. Hierzu können einfache Vorversuche durchgeführt werden. Die Reaktionszeit kann sinnvollerweise zwischen 15 Minuten und 2 Stunden liegen. Die Reaktion könnte auch kontinuierlich in einem Durchlaufofen gefahren werden.

Versuche haben ergeben, dass ein Umsatz von 95 bis 97 % bezogen auf den Säureverbrauch innerhalb praktikabler Reaktionszeiten von nicht mehr als einigen Stunden möglich ist.

Ein erheblicher Vorteil des Verfahrens liegt darin, dass direkt, ohne dass weitere Verfahrensschritte wie Kristallisation, Sprühtrocknung, Filtration erforderlich wären, ein unmittelbar als Futtermittel-Zusatzstoff einsetzbares Produkt entsteht. Dieses Produkt ist aus industriell verfügbaren, relativ preiswerten Ausgangsstoffen erhältlich.

Das Zinkoxid kann im stöchiometrischen Verhältnis zur Säure, aber auch bewusst im Überschuss eingesetzt werden.

Die Erfindung umfasst ein zinkhaltiges Nahrungsergänzungsmittel für Mensch und Tier gemäß Anspruch 1 sowie die Verwendung von Zinkoxid in Verbindung mit wenigstens einem Aminosäure-Zink-2:1-Komplex in einem Nahrungs- oder Futter-Ergänzungsmittel gemäß Anspruch 3.

Da Zinkoxid selbst in großem Umfang als Futtermittelzusatzstoff verwendet wird, ergibt sich bei Einsatz des Zinkoxids im Überschuss immer ein sinnvolles Futterergänzungs- oder -zusatzmittel. Zinkoxid wird außer als Spurenelement in der Tierernährung auch zur Darmpflege gegeben. Eine Kombination aus ZnO mit einem organisch gebundenen Zinksalz bzw. -chelat wird daher in vielen Fällen zweckmäßig und wünschenswert sein. Die unterschiedlichen Freisetzungswege und Freisetzungskinetiken von ZnO und ZnX₂, wobei X für die im Rahmen dieser Erfindung verwendeten organischen Säuren steht, erleichtern die optimale Verteilung des Zinks im Körper, so dass sich hieraus ein weiterer Zusatznutzen ergibt.

Wird das Zinkoxid im Rahmen der Erfindung mit Aminosäuren umgesetzt, ist praktisch jedes Mischungsverhältnis von ZnO und Aminosäure möglich. Erhalten wird ein Gemisch aus restlichem bzw. überschüssigem Zinkoxid, dem gebildeten Chelat und restlicher bzw. überschüssiger Aminosäure. Die Verfahrensbedingungen können so eingestellt werden, dass ein für den jeweiligen Anwendungszweck optimales Produkt erhalten wird.

Die Reaktion zwischen der organischen Säure und dem Zinkoxid findet an der Oberfläche der Zinkoxid-Teilchen statt. Aufgrund der besonderen Verfahrensführung bei der Erfindung entsteht daher die erfindungsgemäße Organozinkverbindung, d.h. das erfindungsgemäße Chelat (sofern nicht im Einzelfall eine eher als Salz zu bezeichnende Verbindung vorliegt), physikalisch assoziiert mit einem Zinkoxid-Teilchen, bzw. physikalisch in dieses eingebunden oder daran anhaftend. Für den Fall, dass die Umsetzung nicht stöchiometrisch und praktisch vollständig erfolgt, enthält demnach die zinkhaltige Zusammensetzung das ZnO und die organisch koordinierte oder substituierte Zinkverbindung wenigstens teilweise physikalisch miteinander assoziiert. Gegenüber einer Mischung aus Zinkoxid und einem anderen Zinksalz ergibt sich der zusätzliche Vorteil, dass sich dieses Produkt weniger oder nicht entmischen wird.

Vorzugsweise enthält die für die Nahrungsergänzung von Mensch und Tier verwendete zinkhaltige Zusammensetzung neben Zinkoxid eine der folgenden Komplexverbindungen: binäres Zinkmethionat (Zn(Met)2), insbesondere bis(DL-methioninato-N,O)-Zink oder bis(L-methioninato-N,O)-Zink, binäres Zinklysinat (Zn(Lys)2), insbesondere bis(DL-lysinato-N,O)-Zink oder bis(L-lysinato-N,O)-Zink, Zinkmethionatcysteinat (Zn(Met)(Cys)), insbesondere (DL-methioninato-N,O)-DL-cysteinato-N,O-Zink.

Im Folgenden wird die Erfindung anhand von Beispielen und analytischen Untersuchungen näher dargestellt. Die Beispiele dienen allein illustrativen Zwecken, ohne Beschränkung der Allgemeingültigkeit der oben dargestellten Erfindungsprinzipien.

### BEISPIELE

### 1. Allgemeine Daten zu Geräten und Reaktionsansätzen

Die Mikrowellen-gestützten Synthesen wurden durchgeführt in einem PC-gesteuerten Labormikrowellensystem "ETHOS 1600" der Firma MLS (Leutkirch) mit 1600 Watt maximaler Leistung und 2,45 GHz Mikrowellenfrequenz.

Die im Folgenden genannten Reaktionsdaten beziehen sich auf 2,0 g- bzw. 4,5 g-Ansätze von 1:1 Mischungen (Molverhältnis) der Reaktanden Zinkoxid und Säure. Diese wurden in zylindrischen Reaktionsgefäßen mit einem Innendurchmesser von 10 mm umgesetzt. Der unterstöchiometrische Anteil an ZnO wurde in Abstimmung auf den mittleren Teilchendurchmesser des eingesetzten Zinkoxids gewählt, der ca. 1 µm betrug (mittlerer Teilchendurchmesser bestimmt durch Lichtstreuungsverfahren, entsprechend einer spezifischen Oberfläche (BET-Oberfläche) von ca. 4m²/gr), da die Umsetzbarkeit des Zinkoxids grundsätzlich beschränkt ist, weil die Reaktion nur an der Oberfläche der Zinkoxidteilchen stattfindet. Nicht umgesetztes Zinkoxid zeigt sich in den IR-Spektren der Produkte mit einer Bande bei etwa 440 cm-1.

### 2. Experimentelle Ergebnisse

### 2.1. Vorversuche

Für einen Ansatz aus Zinkoxid und Methionin wurden Vorversuche zur Zersetzung des Methionins unter Mikrowellenbedingungen durchgeführt. Oberhalb von etwa 185 °C (gemessen mit zentral in der Reaktionsmischung angeordnetem Messfühler) trat merkliche Zersetzung des organischen Materials ein. Die Reaktionsmischung verfärbte sich dabei beige bis schwach bräunlich. Es traten flüchtige schwefelhaltige Anteile auf. Die Reaktionstemperatur wurde für die weiteren Versuchsreihen auf maximal 175 °C begrenzt.

Nasschemische Gewinnung von Methionin-Zink 1:1-Komplexen (Referenz) 68,0 g Zinkchlorid werden in 68,0 g Wasser aufgelöst, wobei die Lösung auf 90 °C erhitzt wird. Dann werden 74,6 g Methionin zugegeben, worauf die Temperatur 1 h auf 90 °C gehalten wird, um eine Zinkmethioninchlorid Lösung herzustellen (Vorschrift nach DE 24 36 946 A1). Das Produkt kann einem Sprühtrockner zugeführt werden, um unter Gewinnung eines 1:1 Zink-Methioninkomplexes sprühgetrocknet zu werden (nach DE 38 12 653).

### Nasschemische Gewinnung von Methionin-Zink 2:1-Komplexen (Referenz)

0,298 g Methionin und 0,136 g Zinkchlorid werden in 50 ml warmem Wasser gelöst, von einer geringen Trübung abfiltriert und tropfenweise mit n/5 Kalilauge versetzt, bis ein pH-Wert von 5,4 erreicht ist. Der schwerlösliche Methionin-Zink-Komplex fällt sofort aus.

Die Komplexbildung kann folgendermaßen beschrieben werden:

2 CH₃SCH₂CH₂CH(NH₂)COOH + Zn²+ + 2 X- → [CH₃SCH₂CH₂CH(NH₂)COO]₂Zn + 2 HX

Bei dem nasschemischen Verfahren muss die entstehende starke Säure abgefangen werden.

### 2.2. Versuchsreihen zu trockener Erwärmung

In zwei Versuchen mit der nachfolgend wiedergegebenen Reaktionsführung wurde reproduzierbar ein nahezu 100 %iger Umsatz bezogen auf den Methionin-Verbrauch erreicht:

### Mikrowellensynthese

| | |
|---|---|
| Mikrowellenleistung: | 650 Watt |
| Aufheizperiode: | Raumtemperatur bis 175 °C innerhalb von 10 min; |
| isotherme Phase: | 30 min bei 175 °C; |
| Abkühlphase: | innerhalb von etwa 15 min von 175 °C auf Raumtemperatur |
| Ausbeute: | 70 bis 95 % Methionin-Umsatz |

Das Gemisch wurde nach Eintauchen des Temperaturfühlers, vor der Reaktion mit einem elektrischen Vibrationserzeuger verdichtet.

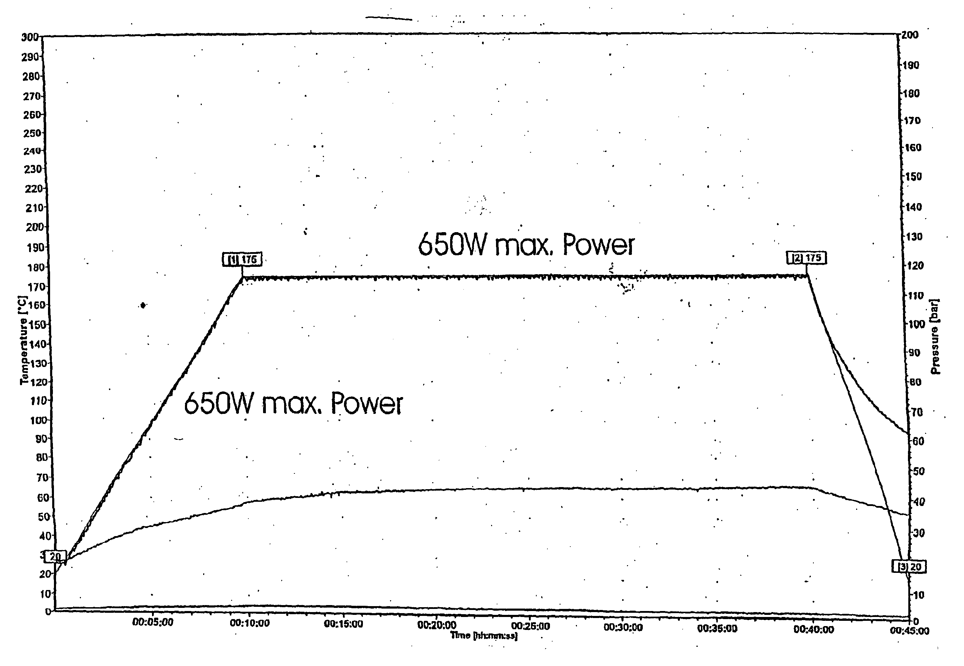

Das Geräteprotokoll mit den IST- und SOLL-Werten der Temperatur der Reaktionsmischung sowie dem Druckverlauf ist in Abb. 1 gezeigt. Die untere Kurve gibt den Druckverlauf wieder, die glatte obere Kurve gibt die Temperatur-SOLL-Werte an, die zweite obere Kurve die Temperatur-IST-Werte.

### 2.3. Analytik

Der analytische Nachweis des gebildeten Zink-Methionins gelingt unter anderem mittels IR-Spektroskopie. Die Carbonylschwingung der Carboxylgruppe (n^{as}_{COO}⁻) des Produktes zeigt bei einer relativ geringfügigen Lageänderung von D =25cm'' eine signifikante Änderung der Signalform gegenüber dem Ausgangsmaterial Methionin: Während das Edukt eine starke Dreifachbande mit Schwerpunkt bei 1581 cm⁻¹ zeigt, ergibt das Produkt Zink-Methionin eine sehr starke Einfachbande bei 1606,6 cm⁻¹ mit leichter Rechtsschulter.

Auch die symmetrische Carboxylat-Streckschwingung hat in Edukt und Produkt eine unterschiedliche Lage und ist daher analytisch nutzbar: Während diese in Methionin bei 1413,7 cm⁻¹ erscheint, liegt sie im Produkt bei 1425,3 cm⁻¹. Vergleichbare Wellenzahlendifferenzen zwischen L-a-Methionin und Zinkkomplexen des Methionins sind bekannt (J. Liu et al., J. Therm. Anal. Cal. 1999, 58, 323).

Die ¹³C-NMR-spektroskopische Untersuchung (mit NOE-Verstärkung durchgeführt) zeigt eine deutlichere Signaltrennung. Dies ermöglicht auch die Anteilsbestimmung Produkt/Edukt über die Signalfläche. Das Carboxylkohlenstoff-Signal des Eduktes findet sich bei 177,2 ppm und damit etwa 11 ppm abgeschirmt ("rechts") vom Produktsignal bei 188.8 ppm (Werte bezogen auf den internen Standard TMSP-d₄ = 0,0 ppm).

Eine schnell nutzbare Aussage über den Reaktionsfortschritt ergibt sich außerdem aus dem Vorhandensein eines starken endothermen Peaks bei 350 °C in der thermischen Analyse mittels DSC (Differential Scanning Calorimetry). Während dieser auf das Produkt hinweist, gibt sich restliches Methionin durch ein nur schwach ausgeprägtes endothermes Signal nahe 280 °C zu erkennen. Eine Abschätzung des Methionin-Verbrauchs ist mittels einer Kalibrierreihe für das 280 °C-Signal möglich.

## Patentansprüche

1. Zinkhaltiges Nahrungsergänzungsmittel für Mensch und Tier, enthaltend eine wenigstens vierfach organisch koordinierte oder zweifach organisch substituierte Zinkverbindung und einen Anteil an Zinkoxid, wobei die organisch koordinierte oder substituierte Zinkverbindung wenigstens teilweise mit dem Zinkoxid (ZnO) physikalisch assoziiert, d.h. physikalisch in das Zinkoxid eingebunden oder daran anhaftend, vorliegt.

2. Nahrungsergänzungsmittel nach Anspruch 1, **dadurch gekennzeichnet, dass** die organische Zinkverbindung eine der folgenden Komplexverbindungen ist:
binäres Zinkmethionat, insbesondere bis(DL-methioninato-N,O)-Zink oder bis(L-methioninato-N,O)-Zink, binäres Zinklysinat, insbesondere bis(DL-lysinato-N,O)-Zink oder bis(L-lysinato-N,O)-Zink, Zinkmethionatcysteinat, insbesondere (DL-methioninato-N,O)-DL-cysteinato-N,O-Zink.

3. Verwendung von Zinkoxid in Verbindung mit wenigstens einem Aminosäure-Zink 2:1-Komplex in einem Nahrungs- oder Futter-Ergänzungsmittel, wobei die organisch koordinierte oder substituierte Zinkverbindung wenigstens teilweise mit dem Zinkoxid (ZnO) physikalisch assoziiert, d.h. physikalisch in das Zinkoxid eingebunden oder daran anhaftend, vorliegt.

## Claims

1. Zinc-containing food supplement for humans and animals, comprising an at least four-fold organically coordinated or organically disubstituted zinc compound and a fraction of zinc oxide, the organically coordinated or substituted zinc compound being present at least in part physically associated with the zinc oxide (ZnO), i.e. physically incorporated into the zinc oxide or adhering thereto.

2. Food supplement according to Claim 1, **characterized in that** the organic zinc compound is one of the following complex compounds:
binary zinc methionate, in particular bis(DL-methioninato-N,O)zinc or bis(L-methioninato-N,O)-zinc, binary zinc lysinate, in particular bis(DL-lysinato-N,O)zinc or bis(L-lysinato-N,O)zinc, zinc methionate cysteinate, in particular (DL-methioninato-N,O)-DL-cysteinato-N,O-zinc.

3. Use of zinc oxide in combination with at least one amino acid-zinc 2:1 complex in a food supplement or feed supplement, the organically coordinated or substituted zinc compound being present at least in part physically associated with the zinc oxide (ZnO), i.e. physically incorporated into the zinc oxide or adhering thereto.

## Revendications

1. Complément alimentaire contenant du zinc pour l'homme et l'animal, contenant un composé du zinc organiquement au moins tétracoordiné ou au moins organiquement disubstitué et une proportion d'oxyde de zinc, le composé du zinc organiquement coordiné ou substitué se trouvant au moins partiellement sous une forme physiquement associée avec l'oxyde de zinc (ZnO), c'est-à-dire incorporé physiquement dans l'oxyde de zinc ou adhérant à celui-ci.

2. Complément alimentaire selon la revendication 1, **caractérisé en ce que** le composé organique du zinc est un des composés complexes suivants : méthionate de zinc binaire, en particulier bis(DL-méthioninato-N,O)-zinc ou bis(L-méthioninato-N,O)-zinc, lysinate de zinc binaire, en particulier bis(DL-lysinato-N,O)-zinc ou bis(L-lysinato-N,O)-zinc, méthionate-cystéinate de zinc, en particulier (DL-méthioninato-N,O)-DL-cystéinato-N,O-zinc.

3. Utilisation d'oxyde de zinc en association avec au moins un complexe 2:1 acide aminé-zinc dans un complément alimentaire ou complément fourrager, le composé du zinc organiquement coordiné ou substitué se trouvant au moins partiellement sous une forme physiquement associée avec l'oxyde de zinc (ZnO), c'est-à-dire incorporé physiquement dans l'oxyde de zinc ou adhérant à celui-ci.
